# EUROPÄISCHE PATENTSCHRIFT

(11) **EP 3 284 516 B1**
(45) Veröffentlichungstag und Bekanntmachung des Hinweises auf die Patenterteilung: **02.10.2019**
(21) Anmeldenummer: 17184616.5
(22) Anmeldetag: 03.08.2017
(51) Int. Cl.: A61N 1/375, A61N 1/05, A61N 1/02

(54) **IMPLANTAT UND VERFAHREN ZUR IDENTIFIKATION EINER ELEKTRODENLEITUNG**
IMPLANT AND METHOD FOR THE IDENTIFICATION OF AN ELECTRODE LEAD
IMPLANT ET PROCÉDÉ D'IDENTIFICATION D'UNE LIGNE D'ÉLECTRODES

(30) Priorität: 16.08.2016 EP 16184299; 16.08.2016 EP 16184298; 16.08.2016 EP 16184297
(43) Veröffentlichungstag der Anmeldung: 21.02.2018
(73) Patentinhaber: BIOTRONIK SE & Co. KG, 12359 Berlin (DE)
(72) Erfinder: RUMP, Jens, 12049 Berlin (DE)
(74) Vertreter: Keck, Hans-Georg

(56) Entgegenhaltungen:
- WO-A1-2011/093875
- DE-A1-102008 040 867
- US-A1- 2007 203 547
- US-A1- 2014 330 347
- US-A1- 2014 343 633
- US-B2- 7 983 763

## Beschreibung

Die Erfindung betrifft eine Elektrodenleitung zur Verbindung mit einem Implantat mittels eines Steckers mit mindestens einem elektrischen Leiter und einem den mindestens einen elektrischen Leiter isolierenden Isolationsschlauch, ein Verfahren zur Identifikation einer Elektrodenleitung sowie ein Implantat mit einer Buchse zur Verbindung mit dem Stecker einer Elektrodenleitung.

Implantate (implantierbare Medizingeräte, IMD) wie Herzschrittmacher (Pacemaker), Defibrillatoren oder neurologische Geräte wie Himschrittmacher für tiefe Hirnstimulation (Deep Brain Stimulation), Rückenmarkstimulationsgeräte, TENS (Transcutaneous Electrical Nerve Stimulator), Geräte für muskuläre Stimulationstherapie, oder Diagnosegeräte, welche die chemischen Eigenschaften des Blutes des Patienten, anderer Körperteile oder andere Körpereigenschaften und -parameter untersuchen, verwenden häufig Elektrodenleitungen, die in den Körper des Patienten geführt werden und dort zumindest über den Zeitraum der Behandlung oder Messung verbleiben. Die Elektrodenleitungen sind mit dem Implantat elektrisch leitend verbunden.

Die Implantate umfassen üblicherweise ein körperverträgliches Gehäuse mit einer dazugehörigen elektronischen Schaltung und einer Energieversorgung, z. B. einer Batterie. Das Gehäuse besitzt mindestens eine Buchse, an der eine oder mehrere Elektrodenleitungen angeschlossen werden können, beispielsweise mittels eines Steckers. Eine Elektrodenleitung dient der Übertragung der elektrischen Energie bzw. von Daten vom Gehäuse zu dem behandelten oder untersuchten Körperteil und umgekehrt.

Im Rahmen der vorliegenden Erfindung wird unter dem Begriff "Elektrodenleitung" eine Leitung mit einem elektrischen Leiter oder mehreren elektrischen Leitern zusammen mit dem umhüllenden, den oder die elektrischen Leiter nach außen und ggf. gegeneinander elektrisch isolierenden Isolationsschlauch sowie allen weiteren funktionellen Elementen, die mit der Leitung fest verbunden sind, verstanden. Die Elektrodenleitung umfasst in der Regel an ihrem distalen Ende auch eine sogenannte Elektrodenspitze, mittels der die elektrische Energie von dem oder den Leiter(n) in das zu behandelnde Gewebe eingeleitet wird. Häufig ist eine Elektrodenspitze mit Ankerelementen oder Haltestrukturen versehen, mit denen die Konstanz der räumlichen Lage der Übergangsstelle der elektrischen Energie in das zu behandelnde Gewebe sichergestellt wird. Die Elektrodenspitze kann als Ableit-, Stimulations- oder Messelektrode ausgebildet sein. Weiter besitzt die Elektrodenleitung in der Regel, beispielsweise an ihrem proximalen Ende, einen Stecker, mit der die Elektrodenleitung mit einem Implantat verbunden werden kann, wobei der Stecker hierfür in eine entsprechende Buchse des Implantats eingesteckt wird. Der Stecker besitzt einen oder mehrere Anschlüsse, wobei jeder Anschluss mit genau einem elektrischen Leiter der Elektrodenleitung verbunden ist. Entsprechend ist in der Buchse für jeden Anschluss der Elektrodenleitung ein Anschluss der Buchse vorgesehen.

An moderne Implantate, beispielsweise einen Mehrkammer-Herzschrittmacher, werden häufig mehrere Elektrodenleitungen angeschlossen. Hierbei besteht das Bestreben, die Elektrodenleitungen und ihre Anschlüsse möglichst dünn bzw. klein zu gestalten. Dies erschwert jedoch die gut sichtbare Markierung der Stecker und Anschlüsse sowie die Unterscheidung der Elektrodenleitungen. Darüber hinaus steigt mit zunehmender Anzahl von Elektrodenleitungen die Gefahr, dass einzelne Elektrodenleitungen verwechselt und/oder falsch angeschlossen werden. Deshalb ist es wünschenswert, wenn ein Implantat erkennt, welche Elektrodenleitungen angeschlossen sind, so dass es diese adäquat ansteuern kann. Zudem ist es zum Betreiben des Implantats hilfreich, wenn die Elektrodenleitungen und/oder ihre Eigenschaften für das Implantat identifizierbar sind.

Dokument US 2004/0073265 A1 beschreibt eine Vorrichtung, die eine Möglichkeit bietet, falsch verbundene Koronarleitungen und/oder falsche Verbindungen zu Herzrhythmusmanagement-Vorrichtungen zu erkennen. Dazu erzeugt eine Spannungseinbringungsvorrichtung eines Schrittmachers einen Spannungspuls zwischen einer Elektrode, die mit einer Leitung mit dem Schrittmacher verbunden ist, und einer Kopf- oder Gehäuseelektrode des Schrittmachers. Die Gehäuseelektrode sendet ein Verbindungssignal. Die Elektrode wird genutzt, um ein korrespondierendes Verbindungssignal unter Nutzung der Leitung zu messen. Ein Messmodul der Vorrichtung misst weiter eine oder mehrere Eigenschaften des korrespondierenden Verbindungssignals wie seine Stromstärke, Spannung, Impedanz und/oder sein Zeitverzug (nach Aussendung des Spannungspulses). Die Signaleigenschaften können von einer oder mehreren Leitungen und/oder vom übermittelnden Gewebe und Flüssigkeiten (beispielsweise einem Herz inklusive einer oder mehrere seiner Kammern), die dazwischen liegen, beeinflusst werden. Ein Vergleichsmodul des Schrittmachers kann daraufhin feststellen, ob die Leitung ordnungsgemäß zu einem Kontakt des Schrittmachers geführt ist, wobei eine oder mehrere Eigenschaften des korrespondierenden Verbindungssignals mit geeigneten vorgewählten Wertebereichen verglichen werden. Beispielsweise kann eine gemessene Impedanz mit einem erwarteten Impedanzbereich verglichen werden. Die in der Druckschrift beschriebene Vorrichtung identifiziert damit nicht selektiv die Leitung, sondern testet vielmehr, ob das korrespondierende Vergleichssignal, das über eine Leitung nach Anregung des Körpers durch einen Spannungspuls einer Gehäuseelektrode des Schrittmachers empfangen wird, Eigenschaften innerhalb eines vorgegebenen Wertebereichs aufweist. Die Eigenschaften des korrespondierenden Vergleichssignals werden auch durch das angeregte Körpergewebe zwischen der Gehäuseelektrode des Schrittmachers und der empfangenden Elektrode bestimmt. Nur sehr grobe Abweichungen, wie sie durch eine nicht verbundene oder vollkommen falsche Art von Leitung auftreten, können sicher auf die Leitung zurückgeführt werden, geringere Abweichungen können körperbedingt sein. Die oben genannte Vorrichtung kann damit die sichere und gezielte Erkennung und Unterscheidung von Elektrodenleitungen mit ähnlichen Eigenschaften nicht gewährleisten.

Eine ähnliche Vorrichtung wird auch in Dokument US 2006/0212083 A1 offenbart. Auch in dieser Druckschrift wird betont, dass die Signaleigenschaften von den Leitungen und/oder von dem/der dazwischen liegenden übermittelnden Gewebe und Flüssigkeit beeinflusst werden.

Dokument US 2011/0112609 A1 beschreibt ein System zur Rückenmarksstimulation mit mindestens einer implantierbaren Stimulationsleitung. Es umfasst insbesondere ein ärztliches Programmiergerät und einen implantierbaren Pulsgenerator, der mit einer oder mehreren implantierbaren Stimulationsleitungen verbunden ist, die je eine Vielzahl von Elektroden tragen. Die Stimulationsleitung weist ein oder zwei Leitungskörper auf. Die Elektroden passen genau in den Epiduralraum der Wirbelsäule. Da das dortige Gewebe leitend ist, können elektrische Messungen zwischen den Elektroden durchgeführt werden. Ein Kontrollschaltkreis des implantierbaren Pulsgenerators erfasst solche elektrische Messungen, so dass das ärztliche Programmiergerät automatisch die einzelnen Leitungskörper identifizieren kann, die mit dem implantierbaren Pulsgenerator verbunden sind. Die elektrischen Messungen des Kontrollschaltkreises zur Identifikation der verbundenen Leitungskörper sind Feldpotentiale. Der Kontrollschaltkreis kann auch die Impedanz an jeder Elektrode messen, um die Kopplungseffizienz zwischen der jeweiligen Elektrode und dem Gewebe zu bestimmen und um die Fehlerkennung bezüglich der Verbindung zwischen der Elektrode und dem analogen Ausgabeschaltkreis des implantierbaren Pulsgenerators zu bestimmen. Bei dem bekannten System ist von Nachteil, dass die Identifikation nicht durch den implantierbaren Pulsgenerator selbst, sondern durch ein zusätzliches ärztliches Programmiergerät erfolgt.

Dokument US 2012/0123496 A1 beleuchtet die Erkennung der Verbindung und die Identifikation des Typs einer implantierten Leitung für eine implantierte medizinische Vorrichtung. Die Vorrichtung weist einen Prozessor auf, der die Verbindung sowie den Leitungstyp der Leitung bestimmen kann. Zunächst prüft ein Signalmessmodul die Verbindung der Leitungen, indem es Werte elektrischer Parameter während eines Signals zwischen zumindest zwei Elektroden prüft, insbesondere der Impedanz. Eine oder mehrere Leitungen können darin integrierte, aktive Elektronik aufweisen, die einen oder mehrere darin integrierte modulare Schaltkreise beinhaltet, je nachdem, ob die Leitung unipolar oder multipolar ist. Jeder der modularen Schaltkreise ist in der Lage, eine Vielzahl von Elektroden der Leitung zu steuern, und schließt eine Schaltungsanordnung ein, die elektrisch mit einer oder mehreren Elektroden der Leitung verbunden ist. Als solcher wirkt jeder der modularen Schaltkreise einer Leitung als Schnittstelle zwischen der implantierten medizinischen Vorrichtung und den Elektroden, mit denen der modulare Schaltkreis verbunden ist. Für die Messung der Impedanz steuert der Prozessor der Vorrichtung den modularen Schaltkreis, sodass dieser einen Spannungspuls zwischen einer ersten und einer zweiten Elektrode liefert. Das Signalmessmodul misst den resultierenden Strom und der Prozessor leitet den Impedanzwert ab. In einem weiteren Schritt sendet der Prozessor ein Abfragesignal entlang eines ersten Leiters der Leitung, um eine Antwort von den modularen Schaltkreisen über eine zweite Leitung zu erhalten. Eine solche Antwort von jedem modularen Schaltkreis liefert dem Prozessor Informationen zu dem modularen Schaltkreis und den Elektroden, die er steuert. In einem weiteren Konfigurationsschritt sendet der Prozessor ein Signal über die erste Leitung. Der Konfigurationsschritt schließt ein, dass die aktive Konfiguration der modularen Schaltkreise programmiert wird. Bezüglich Leitungsausführungen und darin verwendeter aktiver Elektronik bzw. modularer Schaltkreise wird auf das Dokument US 7,713,194 verwiesen. Gemäß dieser Druckschrift ist der modulare Schaltkreis derart ausgestaltet, dass er durch einen Bus gesteuert wird. Die Druckschrift US 2012/0123496 A1 beschreibt demnach, dass die zusätzliche Schnittstellenelektronik modularer Schaltkreise erkannt und somit die Elektrodenleitung bestimmt werden kann. Bei der bekannten Vorrichtung ist nachteilig, dass komplexe modulare Schaltkreise mit aktiver Elektronik zur Steuerung der Elektroden implementiert und programmiert werden müssen. Ferner beziehen sich die Informationen nur auf die modularen Schaltkreise und die damit verbundenen Elektroden, nicht aber auf die Leitung als Ganzes.

Ein Verfahren und eine Vorrichtung zur automatischen Erkennung von implantierbaren medizinischen Leitungen und deren Konfiguration sind in dem Dokument US 2003/0018369 A1 dargestellt. Dafür ist ein erster Kommunikationsschaltkreis, der Daten wie Modell- und Seriennummer, technische Information und Kalibrierungsdaten speichert, mit der Leitung verbunden oder in ihr integriert. Dieser weist einen Empfänger und einen Sender auf, um Datensignale von einer externen Quelle zu empfangen. So kann er bei der Herstellung mit Identifikationsdaten, Kalibrierungsdaten und anderen Daten programmiert werden. Der erste Kommunikationsschaltkreis ist als passiver Transponder ausgeführt und weist neben dem Receiver und Transmitter weiter einen Energiekoppler zur Energieversorgung und einen Steuerschaltkreis auf, welcher mit einem nicht-flüchtigen Speicher verbunden ist. Der Steuerschaltkreis liefert die in dem Speicher gespeicherten Informationen der Leitung zum Transmitter/Receiver des Transponders, der die Daten via RF oder anderer Kommunikation übermittelt. Während der Implantation der Leitung oder danach können die Informationen zu einem zweiten Kommunikationsschaltkreis außerhalb der Leitung transferiert werden. Die transferierten Daten können zur Identifikation der Leitung genutzt werden, in eine Patientenakte aufgenommen und in einen Zentralspeicher für die Nutzung durch Gesundheitsdienstleister transferiert werden. Anhand des Transponders kann die Leitung automatisch erkannt werden und die im Speicher abgelegten Daten können direkt transferiert und weitergegeben werden. Der Transponder benötigt neben einem Transmitter und Receiver allerdings eine separate Energieversorgung, eine Steuereinheit und einen programmierbaren, digitalen Speicher. Dadurch ist der Gesamtaufbau der Leitung vergleichsweise aufwendig und teuer.

Auch Dokument US 2014/0343633 A1 stellt eine elektrisch identifizierbare Elektrodenleitung mit einem Identifikationsmodul dar, welches zumindest einen Filter, einen Stromkonverter, eine Kommunikationsschaltung, einen Lastschalter und eine Speichereinheit wie ein EPROM zur Speicherung eines Identifikationscodes aufweist. Vor der Einbringung des Implantats wird jede Leitung implantiert und mit dem implantierbaren Pulsgenerator (oder einem externen Pulsgenerator) verbunden, welcher dann selbstidentifizierende Daten vom Identifikationsmodul ausliest und diese Information an eine externe Vorrichtung wie den Arztprogrammierer übermitteln kann. Dafür kann das Identifikationsmodul bis zu 32 Byte Daten speichern. Dieses Verfahren wird für jede Leitung, die implantiert wird, wiederholt. Das Identifikationsmodul nutzt zwei vorhandene Kontakte der Leitung zur Verbindung mit dem implantierbaren Pulsgenerator. Wie im zuvor genannten Dokument wird auch für diese bekannte Elektrodenleitung ein digitaler Speicher benötigt und der Aufbau des Identifikationsmoduls ist ähnlich komplex.

Aus den Dokumenten US 2006/0212096 A1, US 2008/0065181 A1 und US 7,983,763 B2 sind Vorrichtungen für die Identifizierung einer implantierbaren medizinischen Einrichtung und eines implantierten Leitersystems bekannt, bei dem ein RFID-Tag mit einem RFID-Chip in der Isolation, welche den Leiter umgibt, oder in dem Header einer implantierbaren Vorrichtung angeordnet ist. Ferner ist eine Leseeinrichtung vorgesehen, welche die in dem RFID-Chip gespeicherten Daten über das Gerät, das Leitersystem, den Hersteller oder den Patienten kabellos auslesen kann. Die auslesbaren Informationen enthalten jedoch keine Angaben über die aktuelle Anordnung und/oder den Anschluss der jeweiligen Elektroden. Zudem ist es bei den in diesen Dokumenten erläuterten Lösungen von Nachteil, dass vergleichsweise viel Energie für das Abfragen der Daten aus dem Implantat und für die Aktivierung des Chips aufgewendet werden muss. Hierfür werden zusätzliche Geräte eingesetzt, die eine erhebliche SAR-Belastung (SAR = specific absorption rate - Maß für die Absorption eines elektromagnetischen Feldes durch das Gewebe) des Patienten darstellen.

Dokument US 2007/203547 A1 beschreibt eine Elektrodenleitung die mit einem drahtlos auslesbaren Element zur Identifikation der Elektrodenleitung ausgestattet ist.

Die Aufgabe der vorliegenden Erfindung besteht deshalb darin, ein einfaches Verfahren zur Identifikation einer Elektrodenleitung anzugeben, welches eine eindeutige Zuordnung einer auf eine Elektrodenleitung bezogene Information zu einer Buchse des Implantats ermöglicht. Die Aufgabe besteht ferner darin, ein entsprechendes Implantat zu schaffen.

Zur Lösung der obigen Aufgabe wird eine Elektrodenleitung mit einem Stecker zur Verbindung mit einem Implantat, das eine Steuereinrichtung und eine mit der Steuereinrichtung verbundene Kommunikationsantenne aufweist benötigt.

Bei der Elektrodenleitung ist ein
▪ in den Isolationsschlauch und/oder
▪ in den Stecker oder
▪ in einen isolierenden Körper eines mit dem Isolationsschlauch oder den Stecker vorzugsweise formschlüssig verbindbaren, separaten Zusatzteils eingebettetes,
hermetisch abgeschlossenes passives RFID-Label vorgesehen, wobei das RFID-Label ein RFID-Inlay mit einem RFID-Chip und eine mit dem RFID-Chip elektrisch leitend verbundene Inlay-Antenne aufweist, wobei die Inlay-Antenne elektromagnetisch mit dem mindestens einen elektrischen Leiter und der Kommunikationsantenne gekoppelt ist.

Durch diese Elektrodenleitung können auf einfache und kostengünstige Weise Informationen die Elektrodenleitung betreffend ausgelesen werden, ohne einen therapeutischen Pfad der Elektrodenleitung durch eine galvanische Kopplung zu beeinflussen. Erfindungsgemäß arbeiten Kommunikationsantenne und elektrischer Leiter der Elektrodeneinrichtung als bipolare Antenne. Hierdurch ist der Leistungsbedarf zum Auslesen der Informationen deutlich reduziert. Hierdurch wird auch die SAR-Belastung des Patienten verringert. Weiter können lediglich RFID-Label abgefragt werden, die sich in unmittelbarer Nähe der Elektrodenleitung befinden, so dass die Abfrage / übersandte Information der Elektrodenleitung an das Implantat hochspezifisch ist. Weiter ist es bei der erfindungsgemäßen Lösung nicht erforderlich, in das Implantat integrierte EMI-Schutzkondensatoren als Filtereinheit gegen elektromagnetische Interferenz (EMI = elektromagnetische Interferenz) während einer solchen Abfrage von Daten der angeschlossenen Elektrodenleitung abzuschalten.

Für den Betrieb des an das Implantat angeschlossenen elektrischen Leiter der Elektrodenleitung sowie der Kommunikationsantenne des Implantats als bipolare Antenne ist es vorteilhaft, wenn der Gangunterschied der Phase zwischen elektrischem Leiter der Elektrodenleitung am Ort des RFID-Label und Kommunikationsantenne zwischen 140° und 220°, weiter bevorzugt zwischen 150° und 210°, weiter bevorzugt zwischen 160° und 200°, weiter bevorzugt zwischen 170° und 190°, weiter bevorzugt 180° beträgt.

Ein RFID-Label umfasst einen RFID-Chip und ein RFID-Inlay, wobei der RFID-Chip auf dem RFID-Inlay angeordnet ist. Das RFID-Inlay seinerseits umfasst ein Substrat auf dem die Inlay-Antenne, optional Vorrichtungen zur Impedanzanpassung und Leiter oder Leiterbahnen zur Kontaktierung des RFID-Chip mit der Inlay-Antenne und der/den optionalen Vorrichtungen zur Impedanzanpassung angeordnet sind.

Das passive RFID-Label kann entweder in den Isolationsschlauch und/oder in den Stecker eingebettet sein. Alternativ kann ein separates Bauteil, nämlich ein sogenanntes Zusatzteil vorgesehen sein, in dessen isolierenden Körper das passive RFID-Label hermetisch abgeschlossen eingebettet sein kann. Das Zusatzteil ist mit dem Isolationsschlauch oder dem Stecker vorzugsweise formschlüssig verbindbar, beispielsweise auf diese aufsteckbar, ausgebildet. Der isolierende Körper des RFID-Label kann beispielsweise aus dem gleichen Material wie der Isolationsschlauch oder der Stecker bestehen. Beispielsweise kann das RFID-Label an der Außenseite des Isolationsschlauchs durch Verwendung einer Isolationshülse als Zusatzteil ausgebildet sein, welche den Isolationsschlauch umgibt und das RFID-Label hermetisch abschließt. Hierfür ist das RFID-Label zwischen Isolationsschlauch und Isolationshülse und/oder in der Isolationshülse angeordnet.

In einem bevorzugten Ausführungsbeispiel weist der RFID-Chip eine vorzugsweise mehrfach beschreibbare Speichereinheit zum Speichern von über die Antenne übertragenen Informationen auf, zum Beispiel 512 Bit frei beschreibbar und 240 Bit für den Electronic Product Code (EPC). Zusätzlich können Informationen in der Speichereinheit gespeichert werden, die werksseitig vorgegeben werden. Auch diese Informationen können über die Antenne des RFID-Label und den elektrischen Leiter der Elektrodenleitung an das Implantat übertragen werden. Die in dem Speicher gespeicherten Informationen können beispielsweise umfassen: Hersteller, Typ der Elektrodenleitung, Seriennummer, Herstellungsdatum, Zulassungsregion, Zulassungsbedingungen, Implantationsdatum, Implantationskompatibilität, MRI-Kompatibilität und dergl.. Weiter kann der Speicher Sicherheitsmechanismen und Sicherheitsinformationen enthalten, welche die Integrität (zum Beispiel bei partiellem Datenverlust) und/oder die Echtheit (beispielsweise bei Manipulation) der gespeicherten Informationen sicherstellen beziehungsweise erkennbar machen.

Nach der erfindungsgemäßen Lösung erfolgt das Auslesen des RFID-Chips des RFID-Label vom aktiven Implantat mittels der Kommunikationsantenne sowie mittels des elektrischen Leiters der Elektrodenleitung, welche zusammen als bipolare Antenne betrieben werden. Der elektrische Leiter hat somit eine Doppelfunktion, nämlich seine primäre Aufgabe im Zusammenhang mit der Ableitung von elektrischen Signalen, der Stimulation und/oder der Messung von elektrischen Signalen des Körpers, in den die Elektrodenleitung implantiert ist, und die erfindungsgemäße (sekundäre) Funktion als Komponente der Antenne. Durch die Anordnung der Antenne des RFID-Label in unmittelbarer Nachbarschaft zu dem elektrischen Leiter ist die elektromagnetische Kopplung gut und am RFID-Label verbleibt auch bei geringem Energieaufwand des aktiven Implantats ausreichend Signalstärke zum Auslesen der hiermit übertragenen Informationen. Die Dämpfungsverluste sind gering. Die implantierten Elektrodenleitungen können insbesondere gezielt einzeln ausgelesen werden, was eine Zuordnung der Informationen der elektrischen Elektrodenleitung zur Lage dieser Elektrodenleitung (zum Beispiel atrial, ventrikulär) ermöglicht. Eine galvanische Kopplung von RFID-Label, insbesondere der Inlay-Antenne des RFID-Label, und eines elektrischen Leiters der Elektrodenleitung besteht nicht.

Die Energie, die mittels der bipolaren Antenne in die Antenne des RFID-Label drahtlos eingekoppelt wird, ist hierbei so bemessen, dass bei einer Übertragung dieser Energie auf eine Stelle im Gewebe des Patienten die dadurch auftretenden Temperaturen einen Maximalwert von 47 C nicht überschreiten (Grenzwerte für elektromagnetische Strahlung, die in den menschlichen Körper abgegeben werden darf, finden sich z. B. in: DIN EN 62209-1:2007-03 oder VDE 0848-209-1:2007-03). Zudem sind die verwendeten Amplituden stets kleiner als die im jeweiligen, für die Informationsübertragung genutzten Frequenzbereich (zum Beispiel 840 MHz bis 960 MHz) geltenden Grenzwerte für ungewollte Stimulation. Zur Bewertung der elektromagnetischen Verträglichkeit von Implantaten zur Behandlung von Tachikardien, Bradykardien oder von Geräten für die kardiale Resynchronisationstherapie sei auf ANSI/AAMI/ISO 14117/Ed.l verwiesen.

In einem bevorzugten Ausführungsbeispiel weist der RFID-Chip eine vorzugsweise mehrfach beschreibbare Speichereinheit zum Speichern von über die Antenne übertragenen Informationen auf, zum Beispiel 512 Bit frei beschreibbar und 240 Bit für den Electronic Product Code (EPC). Zusätzlich können Informationen in der Speichereinheit gespeichert werden, die werksseitig vorgegeben werden. Auch diese Informationen können über die Antenne des RFID-Label und die bipolare Antenne, bestehend aus der Kommunikationsantenne und den elektrischen Leiter der Elektrodenleitung an das Implantat übertragen werden. Die in dem Speicher gespeicherten Informationen können beispielsweise umfassen: Hersteller, Typ der Elektrodenleitung, Seriennummer, Herstellungsdatum, Zulassungsregion, Zulassungsbedingungen, Implantationsdatum, Implantationskompatibilität, MRI-Kompatibilität und dergl. Weiter kann der Speicher Sicherheitsmechanismen und Sicherheitsinformationen enthalten, die die Integrität (zum Beispiel bei partiellem Datenverlust) und/oder die Echtheit (beispielsweise bei Manipulation) der gespeicherten Informationen sicherstellen beziehungsweise erkennbar machen.

Es ist weiter von Vorteil, dass die Antenne des RFID-Label als auf einer Kunststoff-Folie angeordnete metallische Schicht ausgebildet ist, welche vorzugsweise mindestens ein Metall der Gruppe enthaltend Platin, Gold, Platinlegierungen und Goldlegierungen, aufweist. Eine derartige Antenne ist einfach und kostengünstig realisierbar. Weiterhin kann eine derartige Antenne sehr einfach an der Elektrodenleitung angebracht werden.

In einem weiteren Ausführungsbeispiel ist das RFID-Label so ausgebildet, dass das komplex konjugierte der Impedanz des RFID-Chips der Impedanz des RFID-Inlays entspricht, wobei das RFID-Inlay eine Inlay-Antenne und optional eine Vorrichtung zur Impedanzanpassung des RFID-Inlays umfasst. Es ist weiter von Vorteil, wenn die Inlay-Antenne des RFID-Label an die Struktur des elektrischen Leiters der Elektrodenleitung angepasst ist, beispielsweise kann die Inlay-Antenne langgestreckt ausgeführt sein wenn der elektrische Leiter der Elektrodenleitung als ein langgestreckter Leiter wie beispielsweise ein Seilzugleiter ausgeführt ist oder die Inlay-Antenne kann beispielsweise als Helix ausgeführt sein, wenn der elektrische Leiter der Elektrodenleitung beispielsweise als ein gewendelter Leiter ausgeführt ist, wobei vorzugsweise der windungssinn der Helixförmigen Inlay-Antenne dem Windungssinn des gewendelten Leiters entspricht.

In einem weiteren Ausführungsbeispiel ist der mindestens eine elektrische Leiter und/oder die Anordnung der Antenne des RFID-Label derart eingerichtet beziehungsweise konzipiert, dass an der Stelle der elektromagnetischen Kopplung der Inlay-Antenne mit dem mindestens einen elektrischen Leiter in einem vorgegebenen Frequenzbereich ein Strom- oder Spannungsbauch der Schwingung erzeugbar ist. Dies ermöglicht eine Verbesserung der magnetischen beziehungsweise elektrischen Kopplung zwischen der Antenne des RFID-Label und des als Komponente der bipolaren Antenne wirkenden elektrischen Leiters. Hierfür kann die Elektrodenleitung entweder hinsichtlich ihrer Abmessungen und/oder elektrischen Parameter schwingungsgünstig gestaltet und/oder der elektrische Leiter implantatsseitig so abgeschlossen werden, das heißt mit einem entsprechenden Impedanzwert versehen werden, dass sich das gewünschte Schwingungsverhalten in dem vorgegebenen Frequenzbereich ergibt.

Die obige Aufgabe wird ferner durch ein Verfahren zur Identifikation einer oben beschriebenen Elektrodenleitung mittels eines Implantats mit einer Steuereinrichtung und einer mit der Steuereinrichtung verbundenen Kommunikationsantenne, wobei das Implantat mit der Elektrodenleitung verbunden ist, gelöst, umfassend die folgenden Schritte:
▪ Senden eines elektromagnetischen Abfragesignals durch den auszulesenden elektrischen Leiter und die Kommunikationsantenne, wobei das Abfragesignal derart gestaltet ist, dass die Kommunikationsantenne und der elektrische Leiter zusammen als bipolare Antenne betrieben werden, wobei das Abfragesignal von der mit dem auszulesenden elektrischen Leiter und der Kommunikationsantenne verbundenen Steuereinrichtung erzeugt wird,
▪ Aktivieren des RFID-Label durch Empfangen des Abfragesignals mittels der Inlay-Antenne des RFID-Label und Weiterleiten des empfangenen Abfragesignals an den RFID-Chip,
▪ Verarbeiten des empfangenen Abfragesignals durch den RFID-Chip (200), Erzeugen eines entsprechenden elektromagnetischen Antwortsignals durch den RFID-Chip und Senden des Antwortsignals mittels der Inlay-Antenne des RFID-Label,
▪ Empfangen des Antwortsignals durch die bipolare Antenne bestehend aus der Kommunikationsantenne und dem auszulesenden elektrischen Leiter der Elektrodenleitung und
▪ Verarbeiten des an die Steuereinrichtung weitergeleiteten empfangenen Antwortsignals in der Steuereinrichtung.

Das erfindungsgemäße Verfahren erlaubt auf einfache und kostengünstige Weise eine Erkennung des oder der elektrischen Leiter einer Elektrodenleitung beziehungsweise das Auslesen von Informationen über die Elektrodenleitung. Beispiele derartiger Informationen sind oben angegeben. Wie oben bereits ausgeführt wurde, kann das erfindungsgemäße Verfahren aufgrund der Kopplung der Kommunikationsantenne mit dem elektrischen Leiter der Elektrodenleitung mit einer sehr geringen Energie durchgeführt werden.

In einem bevorzugten Ausführungsbeispiel kann die Erzeugung des Abfragesignals durch die Steuereinrichtung in regelmäßigen und/oder vorgebbaren Zeitabständen und/oder nach dem Auftreten eines vorbestimmten Ereignisses erfolgen. Derartige Ereignisse können z.B. sein: das Anschließen einer Elektrodenleitung oder der Austausch einer Elektrodenleitung, eine jährliche/monatliche Inventarisierung der Implantate über die Fernabfrage eines zentralen Patient Data Center, eine definierte Änderung einer elektrischen Eigenschaft einer Elektrodenleitung (z. B. infolge einer Impedanzänderung).

In einem weiteren bevorzugten Ausführungsbeispiel kann die Erzeugung des Abfragesignals für das RFID-Label durch die Steuereinrichtung in regelmäßigen und/oder vorgebbaren Zeitabständen erfolgen und nach erfolgreicher Erkennung eines neuen RFID-Label eine weitere Aktivität auslösen. Vorzugsweise kann es sich bei einer solchen Aktivität um die Aktivierung eines Detektionsvorgangs handeln, der feststellt an welcher Buchse des Implantats eine Elektrodenleitung angeschlossen ist.

Wenn mehrere Elektrodenleitungen an dem Implantat angeschlossen sind, muss die Information zu jeder Elektrodenleitung der jeweils von der Elektrodenleitung belegten Buchse des Implantats zugeordnet werden. Dies wird besonders einfach dadurch erreicht, dass dann, wenn zwei oder mehr als zwei Elektrodenleitungen an das Implantat angeschlossen sind, wobei von diesen Elektrodenleitungen eine Elektrodenleitung neu an das Implantat angeschlossen ist, d.h. erst zuvor an das Implantat angeschlossen wurde, das Verfahren zur Identifikation für alle angeschlossenen Elektrodenleitungen nacheinander für jede Elektrodenleitung durchgeführt wird. Dies bedeutet, dass das Abfrageverfahren sowohl für die zuvor bereits angeschlossenen Elektrodenleitungen und die neu angeschlossene Elektrodenleitung durchgeführt wird. Anschließend wird das jeweilige Antwortsignal jeder Elektrodenleitung mit den bereits in einer Speichereinrichtung des Implantats gespeicherten Informationen zu Elektrodenleitungen verglichen. Basierend auf dem Ergebnis des Vergleichs erfolgt eine Zuordnung der Information aus dem RFID-Chip des RFID-Label zu der neu angeschlossenen Elektrodenleitung zu der jeweiligen, , mit der neuen Elektrodenleitung verbundenen Buchse des Implantats.

Die obige Aufgabe wird ferner gelöst durch ein Implantat mit einer Buchse zur Verbindung mit dem Stecker einer oben beschriebenen Elektrodenleitung und mit einem Gehäuse, wobei in dem Gehäuse eine Steuereinrichtung angeordnet ist, welche mit einer Kommunikationsantenne verbunden ist. Erfindungsgemäß ist vorgesehen, dass die Steuereinrichtung zur Verarbeitung eines elektromagnetischen Antwortsignals eingerichtet ist, welches von einer bipolaren Antenne, bestehend aus der Kommunikationsantenne und dem elektrischen Leiter der Elektrodenleitung, wobei diese Elektrodenleitung mit ihrem Stecker in die Buchse eingesteckt ist, so dass der elektrische Leiter elektrisch mit der Steuereinrichtung verbunden ist, empfangen und an die Steuereinrichtung weitergeleitet wird. Ein derartiges aktives Implantat ermöglicht die einfache und kostengünstige Identifikation der an das Implantat angeschlossenen Elektrodenleitungen und das Auslesen der die Elektrodenleitung betreffenden Informationen.

Besonders bevorzugt ist die Steuereinrichtung zudem zur Erzeugung eines elektromagnetischen Abfragesignals auch eingerichtet, welches ebenfalls durch eine bipolare Antenne, bestehend aus der Kommunikationsantenne und dem elektrischen Leiter der Elektrodenleitung, wobei diese Elektrodenleitung mit ihrem Stecker in die Buchse eingesteckt ist, so dass der elektrische Leiter elektrisch mit der Steuereinrichtung verbunden ist, versendet wird.

Um die elektrischen Leiter der Elektrodenleitung einzeln ansprechen zu können, ist es von Vorteil, dass die Buchse eine Vielzahl von Anschlüssen zur Verbindung mit der Vielzahl elektrischer Leiter einer Elektrodenleitung oder mehrerer Elektrodenleitungen aufweist und dass eine Sende- und/oder Empfangseinheit der Steuereinrichtung vorgesehen ist, welche über einen Multiplexer mit der Vielzahl von Anschlüssen verbunden ist.

Wie oben bereits dargestellt wurde ist es von Vorteil, dass die Steuereinrichtung derart eingerichtet ist, dass sie das Abfragesignal in regelmäßigen und/oder vorgebbaren Zeitabständen und/oder nach dem Auftreten eines vorbestimmten Ereignisses erzeugt.

In einer Weiterbildung der Erfindung weist das Implantat zudem eine Detektionseinheit auf, welche das Anschließen einer Elektrodenleitung an das Implantat detektiert. Vorzugsweise misst die Detektionseinheit jeweils die Impedanz der Anschlüsse der Buchsen für die Elektrodenleitungen am Implantat, wobei ein sprunghafter Anstieg der Impedanz an einem Anschluss die erfolgreiche Kontaktierung einer Elektrodenleitung an der Buchse des Implantats anzeigt, die diesen Anschluss enthält. Die Detektionseinheit ist zur Generierung eines Triggersignals eingerichtet, welches erzeugt wird, wenn die Detektionseinheit eine neu verbundene Elektrodenleitung detektiert. Das Triggersignal wird zu einem Signaleingang der Steuereinrichtung übertragen, welche daraufhin das Abfrageverfahren des RFID-Label initiiert.

Die Erfindung wird nachfolgend anhand von Ausführungsbeispielen und unter Bezugnahme auf die Figuren erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Fig. 1: ein Ausführungsbeispiel einer erfindungsgemäßen Elektrodenleitung, verbunden mit einem erfindungsgemäßen Implantat, in einer perspektivischen Ansicht von der Seite,

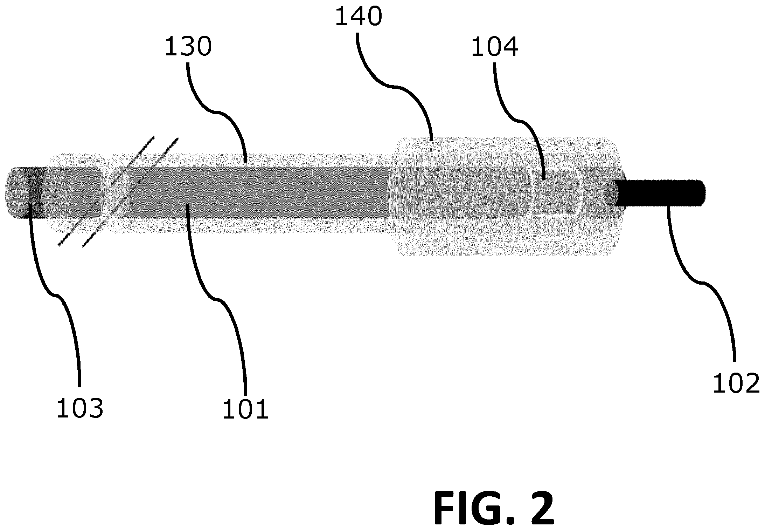

- Fig. 2: eine weitere Darstellung der Elektrodenleitung gemäß Fig. 1 in einer perspektivischen Ansicht von der Seite,
- Fig. 3: eine Schaltskizze des Implantats gemäß Fig. 1 und
- Fig. 4: ein RFID-Label der Elektrodenleitung gemäß Fig. 1,

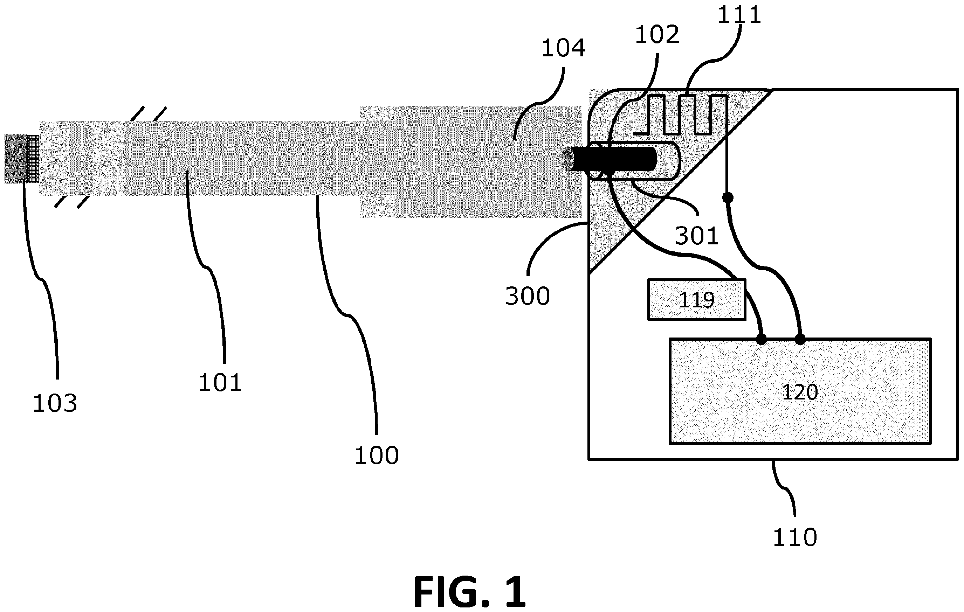

Fig. 1 zeigt ein Ausführungsbeispiel einer erfindungsgemäßen Elektrodenleitung 100 mit einem elektrischen Leiter 101. Alternativ können auch mehrere elektrische Leiter 101 vorgesehen sein. Am distalen Ende der Elektrodenleitung 100 ist eine Elektrodenspitze 103 angeordnet, welche den elektrischen Kontakt zur Umgebung, beispielsweise dem Gewebe des Patienten, herstellt. Die Elektrodenspitze kann als Stimulations-, Mess- oder Ableitelektrode ausgebildet sein. Am proximalen Ende der Elektrodenleitung 100 ist ein Stecker 102 angeordnet, welcher in eine Buchse 301 eines aktiven Implantats 110 eingesteckt ist, welche sich an einem Header 300 des Implantats 110 befindet. Häufig weist das Implantat 110 mehrere Buchsen 301 auf, in die jeweils eine Elektrodenleitung 100 mit ihrem jeweiligen Stecker 102 eingesteckt ist. Das aktive Implantat 110 kann beispielsweise als Herzschrittmacher oder Defibrillator ausgebildet sein. Durch den Stecker 102 besteht eine mechanische und elektrische Verbindung zwischen der Elektrodenleitung 100 und dem aktiven Implantat 110. Über entsprechende Anschlüsse des Steckers 102 beziehungsweise der Buchse 301 besteht insbesondere eine elektrisch leitende Verbindung zwischen dem elektrischen Leiter 101 der Elektrodenleitung 100 und den innenliegenden elektrischen Komponenten des Implantats 110, beispielsweise einer Steuereinrichtung 120.

Zwischen einem beispielsweise als Silikonschlauch ausgeführten Isolationsschlauch 130 und einer am proximalen Ende der Elektrodenleitung 100 in der Nähe des Steckers 102 vorgesehenen Isolationshülse 140 ist ein passives RFID-Label 104 angeordnet. Die Isolationshülse 140 kann als separates Zusatzteil ausgeführt sein, welches über den Isolationsschlauch 130 schiebbar ist. Die Isolationshülse 140, welche beispielsweise aus einem Flüssigkristallpolymer (LCP), Silikon, einer Keramik und/oder Glas besteht, umgibt derart den Isolationsschlauch 130, dass das RFID-Label 104 hermetisch gegenüber der Umgebung abgeschlossen ist.

Das RFID-Label 104 kann alternativ auch in den Stecker 102 integriert sein. Dies hat den Vorteil, dass das RFID-Label 104 nach dem Einstecken des Steckers 102 in die Buchse 301 des Implantats 110 innerhalb des steifen Headers 300 des Implantats 110 angeordnet ist, in dem die Buchse 301 vorgesehen ist, und somit vor hoher mechanischer Belastung (Biegung/Abrieb) geschützt ist. Ein weiterer Vorteil besteht darin, dass eine räumlich nähere Anordnung des RFID-Label 104 in Bezug auf das Implantat 110 geringere Verluste beim Auslesen und damit eine geringere RF-Belastung für den Patienten bedeuten.

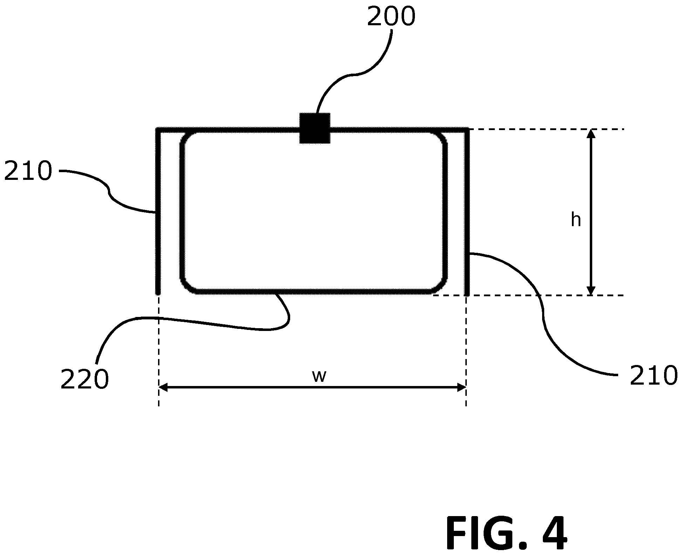

Ein Ausführungsbeispiel für ein RFID-Label 104 ist in Fig. 4 dargestellt. Es ist beispielsweise als ca. 1 cm² großes passives RFID-Label 104 ausgeführt (Abmessungen zum Beispiel Breite w = 16 mm, Länge h = 6 mm, Dicke d < 0,1 mm) und weist einen RFID-Chip 200 sowie eine den RFID-Chip 200 schlaufenförmig umgebende Induktivität 220 zur Impedanzanpassung auf. Ferner ist eine Inlay-Antenne 210 vorgesehen, welche in diesem Ausführungsbeispiel zwei Segmente besitzt. Die Inlay-Antenne 210 kann beispielsweise mittels einer Metallisierung einer Kunststofffolie, vorzugsweise mit Gold oder Platin, hergestellt sein, wobei die Metallisierung auch auf der Innenseite der Isolationshülse 140, beispielsweise einer Keramik- oder Glashülse, angebracht sein kann. Bei dem RFID-Label 104 entspricht das komplex konjugierte der Impedanz des RFID-Chips 200 der Impedanz des RFID-Inlays, Wobei das RFID-Inlay eine Inlay-Antenne 210 und eine Schlaufe 220 zur Impedanzanpassung des RFID-Inlays umfasst.

Als RFID-Chip 200 kann zum Beispiel ein Chip für den Frequenzbereich zwischen 840 MHz und 960 MHz verwendet werden. Weiter kann eine Speicherkapazität von 512 Bit frei beschreibbar und 240 Bit zur Hinterlegung des Electronic Product Code (EPC) vorgesehen sein. Der Speicher des RFID-Chips 200 kann sowohl bei der Herstellung im Werk als auch während der Ausführung des erfindungsgemäßen Verfahrens zur Identifikation einer Elektrodenleitung 100 gelesen und/oder beschrieben werden. Im Speicher des RFID-Chips 200 können Informationen zur Identifikation der Elektrodenleitung 100 sowie zu Ihrer Verwendung enthalten sein. Derartige Informationen können umfassen: den Hersteller, den Typ der Elektrodenleitung, die Seriennummer, das Herstellungsdatum, Zulassungsregionen, Zulassungsbedingungen, Implantationsdatum, Implantationskompatibilitäten, MRI-Kompatibilität und dergleichen. Weiter kann der Speicher des RFID-Chips 200 Sicherheitsmechanismen und Sicherheitsinformationen enthalten, die die Integrität (zum Beispiel bei partiellem Datenverlust) und die Echtheit (zum Beispiel bei Manipulation) der gespeicherten Informationen sicherstellt beziehungsweise erkennbar macht.

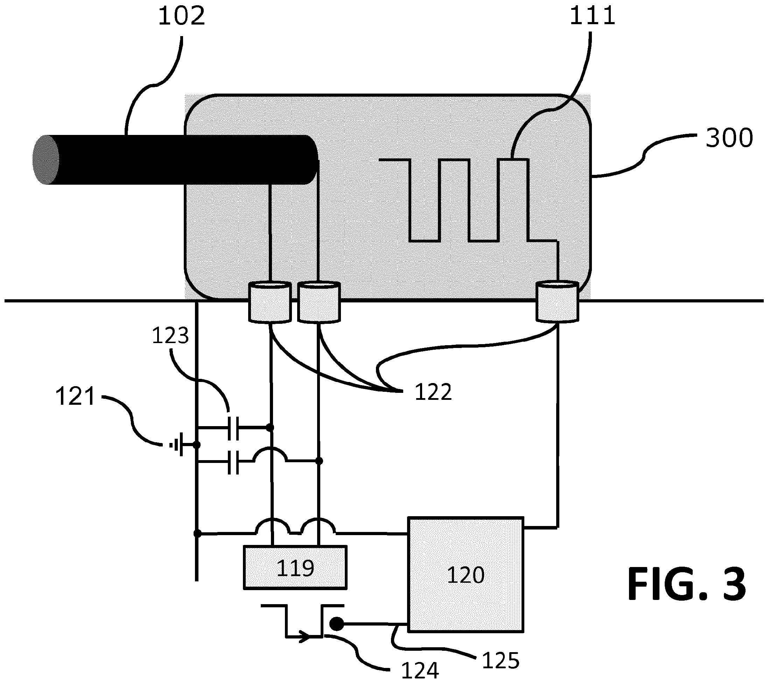

Das Implantat 110 enthält, wie oben bereits erwähnt, eine hermetisch nach außen abgeschlossene Steuereinrichtung 120, welche mit dem Anschluss oder den Anschlüssen in der Buchse 301 für die Elektrodenleitung 100 verbunden ist. Nach Einstecken einer Elektrodenleitung 100 in eine entsprechende Buchse 301 in einem Headerbereich 300 des Implantats 110 (siehe Fig. 3) besteht eine elektrisch leitende Verbindung zwischen der Steuereinrichtung 120 und dem einen elektrischen Leiter 101 oder den mehreren elektrischen Leitern 101 der Elektrodenleitung 100. Diese erfolgt über Durchkontaktierungen 122 in das Innere des gekapselten Implantats 110.

Im Headerbereich 300 des Implantats 110 ist ferner eine Kommunikationsantenne 111 vorgesehen. Die Kommunikationsantenne 111 kann auch als Antenne zur Datenübertragung an einen externen Empfänger verwendet werden, der z. B. Daten an ein Patient Data Center übermittelt oder die Fernabfrage und/oder -programmierung des Implantats 110 ermöglicht. Die Kommunikationsantenne 111 ist über eine Durchkontaktierung (Feedthrough) 122 elektrisch leitend mit der Steuereinrichtung 120 verbunden. Die Kommunikationsantenne 111 weist vorzugsweise für den für die Kommunikation mit dem im RFID-Label 104 eingebetteten RFID-Chip 200 via der Inlay-Antenne 210 vorgesehenen Frequenzbereich eine möglichst gute Anpassung auf. Weiterhin weist die Kommunikationsantenne 111 vorzugsweise eine isotrope Richtcharakteristik auf. Für Antennen 111, die zum Zwecke der Kommunikation zwischen dem Implantat 110 und einem externen Gerät eine nicht isotrope Richtcharakteristik aufweisen, liegt der proximale Bereich der eingesteckten Elektrodenleitung 100 vorzugsweise im bevorzugten Abstrahlbereich der Antenne 111.

Das aktive Implantat 110 besitzt ferner eine Filtereinheit gegen elektromagnetische Interferenz, um beispielsweise das Eindringen von Mobiltelefonsignalen in das Innere des gekapselten Gehäuses des Implantats 110 zu verhindern. Die Filtereinheit schließt hochfrequente Signale (Frequenz > 1 kHz) auf die elektrische Masse 121 kurz. Hierfür werden zum Beispiel parallel geschaltete Kondensatoren 123 mit hoher Kapazität eingesetzt.

Die Kommunikationsantenne 111 wird erfindungsgemäß als bipolare Antenne verwendet, wobei alle vorhandenen elektrischen Leiter 101 aller Elektrodenleitungen 100 und das Gehäuse des Implantats 110 als Gegen-Pol der Kommunikationsantenne 111 genutzt werden. Wenn mittels einer Detektionseinheit 119 des Implantats 110 erkannt wird, dass eine Elektrodenleitung 100 neu an das Implantat 110 angeschlossen wurde, wird durch die Detektionseinheit 119 ein Triggersignal 124 generiert, welches an einen Signaleingang 125 der Steuereinrichtung 120 geleitet wird. Ein möglicher Weg zu erkennen, ob eine Elektrodenleitung 100 mit ihrem Stecker 102 an eine bestimmte Buchse 301 des Implantats 110 angeschlossen wurde, besteht darin, in regelmäßigen Intervallen durch die Detektionseinheit 119 jeweils die Impedanz der Anschlüsse der Buchse 301 für die Elektrodenleitungen 100 am Implantat 110 zu messen, wobei ein sprunghafter Anstieg der Impedanz an einem Anschluss einer Buchse 301 die erfolgreiche Kontaktierung einer Elektrodenleitung 100 an dieser Buchse 301 des Implantats 110 anzeigt. Erkennt die Steuereinrichtung 120 das Triggersignal 124 an seinem Signaleingang 125, so erzeugt die Steuereinrichtung 120 ein hochfrequentes Abfrage-Signal im geeigneten Frequenzbereich (zum Beispiel 860 MHz) und gibt dieses auf den elektrischen Leiter 101 der Elektrodenleitung 100 und die Kommunikationsantenne 111, welche zusammen als bipolare Antenne betrieben werden. In dem Fall, dass zwei oder mehr als zwei Elektrodenleitungen 100 an das Implantat 110 angeschlossen sind, wird das Abfrage-Signal an alle kontaktierten elektrischen Leiter 101 aller angeschlossenen Elektrodenleitungen 100 gleichzeitig gesendet. Für den Betrieb der elektrischen Leiter 101 der an das Implantat 110 angeschlossenen Elektrodenleitungen 100 sowie der Kommunikationsantenne 111 als bipolare Antenne ist es vorteilhaft, wenn der Gangunterschied der Phase zwischen den elektrischen Leitern 101 der Elektrodenleitung 100 und der Kommunikationsantenne 111 etwa 180° beträgt.

Das Abfrage-Signal koppelt in die Inlay-Antenne 210 des jeweiligen RFID-Label 104 der jeweiligen Elektrodenleitung 100 und aktiviert den zugehörigen RFID-Chip 200. Das hierdurch generierte Antwort-Signal des RFID-Label 104 der jeweiligen Elektrodenleitung 100, welches von der zugehörigen Inlay-Antenne 210 versendet wird, wird von der bipolaren Antenne bestehend aus der Kommunikationsantenne 111 und dem elektrischen Leiter 101 der Elektrodenleitung 100 empfangen und an die Steuereinrichtung 120 weitergeleitet. Diese verarbeitet das Antwort-Signal weiter. Durch die räumlich nahe Anordnung zwischen dem hermetisch gekapselten RFID-Label 104, dem elektrischen Leiter 101 und der Kommunikationsantenne 111 ist auch bei nicht optimaler Anpassung eine befriedigende elektromagnetische Kopplung möglich. Elektrische Leiter 101, die sich in unmittelbarer Umgebung des Headerbereichs 300 des Implantats 110 befinden, ohne mit dem aktiven Implantat 110 kontaktiert zu sein, werden entweder nicht oder nur mit einem sehr geringem RSSI (Received Signal Strength Indicator) ausgelesen. Der RSSI wird durch die Steuereinheit 120, z. B. durch Ermittelung des Quotienten der empfangenen mit der Gesendeten Amplitude, ermittelt. Sobald der elektrische Leiter 101 einer Elektrodenleitung 100 mit dem Gegen-Pol der Steuereinrichtung 120 verbunden ist, können die Informationen des jeweiligen RFID-Chips 200 gelesen werden oder der RSSI-Wert des Signals steigt sprunghaft an.

Die Zuordnung der einzelnen Elektrodenleitungen 100 zu den Buchsen 301 des Implantats 110 erfolgt über eine, im Folgenden beschriebene, Logik innerhalb des Implantats 110. Die durch Impedanzmessung erkannte und einer bestimmten Buchse 301 des Implantats 110 zugeordnete neu angeschlossene Elektrodenleitung 100 wird mit Hilfe eines Vergleichs mit dem letzten getriggerten Lesevorgang identifiziert. Eine Speichereinrichtung des Implantats 110 vergleicht hierfür die gespeicherten Informationen (z. B. Elektrodentyp und Anschluss am Implantat 110) der Elektrodenleitungen 100 aus vorherigen Abfrageverfahren mit den empfangenen Antwortsignalen der Elektrodenleitungen 100 und ordnet auf der Basis des Ergebnisses dieses Vergleichs die Informationen zur neu angeschlossenen Elektrodenleitung 100 den entsprechenden Buchsen 301 des Implantats 110 zu.

Mit der erfindungsgemäßen Lösung können spezifische Informationen des elektrischen Leiters 101 einer Elektrodenleitung 100 ausgelesen werden, ohne den therapeutischen Pfad durch eine galvanische Kopplung zu beeinflussen. Der elektrische Leiter 101 einer Elektrodenleitung 100 wirkt als Gegen-Pol, sodass die Informationen erst nach Kontaktierung ausgelesen werden. Der Leistungsbedarf zum Auslesen ist aufgrund der aus elektrischem Leiter 101 und Kommunikationsantenne 111 gebildeten bipolaren Antenne am aktiven Implantat 110 überraschend deutlich reduziert gegenüber einer Abfrage bei der nur die Antenne 111 des Implantats 110 verwendet wird. Es ist nicht notwendig, die EMI-Filtereinrichtung für den Auslesevorgang zu umgehen.

### Bezugszeichenliste

- 100: Elektrodenleitung
- 101: elektrischer Leiter
- 102: Stecker
- 103: Elektrodenspitze
- 104: RFID-Label
- 110: Implantat
- 111: Kommunikationsantenne
- 119: Detektionseinheit
- 120: Steuereinrichtung
- 121: Masse
- 122: Durchführung
- 123: Kondensator der EMI-Filtereinheit
- 124: Triggersignal
- 125: Signaleingang der Steuereinrichtung 120
- 130: Isolationsschlauch
- 140: Isolationshülse
- 200: RFID-Chip
- 210: Inlay-Antenne
- 220: Induktivität zur Impedanzanpassung
- 300: Headerbereich des Implantats 110
- 301: Buchse

## Patentansprüche

1. Verfahren zur Identifikation einer Elektrodenleitung (100) mittels eines Implantats (110),
wobei die Elektrodenleitung (100) einen Stecker (102), mindestens einen elektrischen Leiter (101) und einen den mindestens einen elektrischen Leiter (101) isolierenden Isolationsschlauch (130) umfasst, wobei ein hermetisch abgeschlossenes passives RFID-Label (104) in den Isolationsschlauch (130) und/oder in den Stecker (102) oder in einen isolierenden Körper eines mit dem Isolationsschlauch (130) oder dem Stecker (102) verbindbaren, separaten Zusatzteils eingebettet ist, wobei das RFID-Label (104) einen RFID-Chip (200) und eine mit dem RFID-Chip (200) elektrisch leitend verbundene Inlay-Antenne (210) aufweist, wobei die Inlay-Antenne (210) elektromagnetisch mit dem mindestens einen elektrischen Leiter (101) und der Kommunikationsantenne (111) gekoppelt ist;
wobei das Implantat (110) eine Steuereinrichtung (120) und eine mit der Steuereinrichtung verbundene Kommunikationsantenne (111) aufweist;
wobei das Implantat (110) mit der Elektrodenleitung (100) verbunden ist, umfassend die folgenden Schritte:
- Senden eines elektromagnetischen Abfragesignals durch den auszulesenden elektrischen Leiter (101) und die Kommunikationsantenne (111), wobei die Kommunikationsantenne (111) und der elektrische Leiter (101) zusammen als bipolare Antenne betrieben werden, wobei das Abfragesignal von der mit dem auszulesenden elektrischen Leiter (101) und der Kommunikationsantenne (111) verbundenen Steuereinrichtung (120) erzeugt wird,
- Aktivieren des RFID-Label (104) durch Empfangen des Abfragesignals mittels der Inlay-Antenne (210) des RFID-Label (104) und Weiterleiten des empfangenen Abfragesignals an den RFID-Chip (200),
- Verarbeiten des empfangenen Abfragesignals durch den RFID-Chip (200), Erzeugen eines entsprechenden elektromagnetischen Antwortsignals durch den RFID-Chip (200) und Senden des Antwortsignals mittels der Inlay-Antenne (210) des RFID-Label (104),
- Empfangen des Antwortsignals durch die bipolare Antenne bestehend aus der Kommunikationsantenne (111) und dem auszulesenden elektrischen Leiter (101) der Elektrodenleitung (100) und
- Verarbeiten des an die Steuereinrichtung (120) weitergeleiteten empfangenen Antwortsignals in der Steuereinrichtung (120).

2. Verfahren nach Anspruch 1, **dadurch gekennzeichnet, dass** die Erzeugung des Abfragesignals durch die Steuereinrichtung (120) in regelmäßigen und/oder vorgebbaren Zeitabständen und/oder nach dem Auftreten eines vorbestimmten Ereignisses, vorzugsweise nach dem Anschließen einer Elektrodenleitung (100) an das Implantat (110) erfolgt.

3. Verfahren nach einem der Ansprüche 1 oder 2, **dadurch gekennzeichnet, dass** dann, wenn zwei oder mehr als zwei Elektrodenleitungen (100) an das Implantat (110) angeschlossen sind, wobei von diesen Elektrodenleitungen (100) eine Elektrodenleitung neu an das Implantat (110) angeschlossen ist, das Verfahren zur Identifikation für alle angeschlossenen Elektrodenleitungen (100) nacheinander für jede Elektrodenleitung durchgeführt wird und dass das jeweilige Antwortsignal mit den bereits in einer Speichereinrichtung des Implantats gespeicherten Informationen zu Elektrodenleitungen (100) verglichen und basierend auf dem Ergebnis des Vergleichs eine Zuordnung der Informationen der neu angeschlossenen Elektrodenleitung (100) zu der mindestens einen, mit der neuen Elektrodenleitung (100) verbundenen Buchse (301) des Implantats (110) erfolgt.

4. Implantat (110) mit einer Elektrodenleitung (100),
Wobei die Elektrodenleitung (100) einen Stecker (102), mindestens einen elektrischen Leiter (101) und einen den mindestens einen elektrischen Leiter (101) isolierenden Isolationsschlauch (130) umfasst, wobei ein hermetisch abgeschlossenes passives RFID-Label (104) in den Isolationsschlauch (130) und/oder in den Stecker (102) oder in einen isolierenden Körper eines mit dem Isolationsschlauch (130) oder dem Stecker (102) verbindbaren, separaten Zusatzteils eingebettet ist, wobei das RFID-Label (104) einen RFID-Chip (200) und eine mit dem RFID-Chip (200) elektrisch leitend verbundene Inlay-Antenne (210) aufweist, wobei die Inlay-Antenne (210) elektromagnetisch mit dem mindestens einen elektrischen Leiter (101) und der Kommunikationsantenne (111) gekoppelt ist;
wobei das Implantat (110) weiterhin ein Gehäuse aufweist, wobei in dem Gehäuse eine Steuereinrichtung (120) angeordnet ist, welche mit einer Kommunikationsantenne (111) verbunden ist,
wobei das Implantat (110) eine Buchse (301) aufweist,wobei die Elektrodenleitung (100) mit ihrem Stecker (102) in die Buchse (301) des Implantats (110) eingesteckt ist, so dass der elektrische Leiter (101) elektrisch mit der Steuereinrichtung (120) verbunden ist,
**dadurch gekennzeichnet, dass**
die Steuereinrichtung (120) zur Verarbeitung eines elektromagnetischen Antwortsignals einer bipolaren Antenne, bestehend aus der Kommunikationsantenne (111) und dem elektrischen Leiter (101) der Elektrodenleitung (100) eingerichtet ist.

5. Implantat nach Anspruch 4, **dadurch gekennzeichnet, dass** die Steuereinrichtung (120) zur Erzeugung eines elektromagnetischen Abfragesignals eingerichtet ist, welches durch eine bipolare Antenne, bestehend aus der Kommunikationsantenne (111) und dem elektrischen Leiter (101) der Elektrodenleitung (100), wobei diese Elektrodenleitung (100) mit ihrem Stecker (102) in die Buchse (301) eingesteckt ist, so dass der elektrische Leiter (101) elektrisch mit der Steuereinrichtung (120) verbunden ist, versendet wird.

6. Implantat nach einem der Ansprüche 4 bis 5, **dadurch gekennzeichnet, dass** die Buchse (301) eine Vielzahl von Anschlüssen zur Verbindung mit der Vielzahl elektrischer Leiter (101) einer Elektrodenleitung (100) oder mehrerer Elektrodenleitungen (100) aufweist und dass eine Sende- und/oder Empfangseinheit der Steuereinrichtung (120) vorgesehen ist, welche über einen Multiplexer mit der Vielzahl von Anschlüssen verbunden ist.

7. Implantat nach einem der Ansprüche 4 bis 6, **dadurch gekennzeichnet, dass** die Steuereinrichtung (120) derart eingerichtet ist, dass sie das Abfragesignal in regelmäßigen und/oder vorgebbaren Zeitabständen und/oder nach dem Auftreten eines vorbestimmten Ereignisses erzeugt.

8. Implantat nach Anspruch 7, **dadurch gekennzeichnet, dass** das Implantat (110) eine Detektionseinheit (119) aufweist, welche das Anschließen einer Elektrodenleitung (100) an das Implantat (110) detektiert.

9. Implantat nach Anspruch 7 oder 8, **dadurch gekennzeichnet, dass** das Implantat (110) eine Detektionseinheit (119) aufweist, welche das Anschließen einer Elektrodenleitung (100) an das Implantat (110) durch Messung der Impedanz der jeweiligen Anschlüsse für die Elektrodenleitungen am Implantat detektiert.

## Claims

1. A process to identify an electrode lead (100) by means of an implant (110),
the electrode lead (100) comprising a plug (102), at least one electrical conductor (101), and an insulating tube (130) that insulates the at least one electrical conductor (101), a hermetically sealed passive RFID label (104) being embedded in the insulating tube (130) and/or in the plug (102) or in an insulating body of a separate accessory that is connectable with the insulating tube (130) or the plug (102), the RFID label (104) having an RFID chip (200) and an inlay antenna (210) that has an electrically conductive connection with the RFID chip (200), the inlay antenna (210) being electromagnetically coupled with the at least one electrical conductor (101) and the communication antenna (111),
the implant (110) having a controller (120) and a communication antenna (111) that is connected with the controller,
the implant (110) being connected with the electrode lead (100),
comprising the following steps:
- The electrical conductor (101) to be read out and the communication antenna (111) transmitting an electromagnetic interrogation signal, the communication antenna (111) and the electrical conductor (101) being operated together as a bipolar antenna, the interrogation signal being produced by the controller (120), which is connected with the electrical conductor (101) to be read out and the communication antenna (111);
- Activating the RFID label (104) as a result of the interrogation signal being received by the inlay antenna (210) of the RFID label (104), and forwarding the received interrogation signal to the RFID chip (200);
- The RFID chip (200) processing the received interrogation signal, producing a corresponding electromagnetic response signal, and transmitting this response signal by means of the inlay antenna (210) of the RFID label (104);
- The bipolar antenna receiving the response signal, this bipolar antenna consisting of the communication antenna (111) and that electrical conductor (101) of the electrode lead (100) that is to be read out; and
- Processing, in the controller (120), the received response signal that has been forwarded to the controller (120).

2. the process according to claim 1, **characterized in that** the interrogation signal is produced by the controller (120) at regular and/or specifiable time intervals and/or after the occurrence of a predetermined event, preferably after an electrode lead (100) is connected to the implant (110).

3. The process according to one of the claims 1 or 2, **characterized in that** when two or more than two electrode leads (100) are connected to the implant (110), one of these electrode leads (100) being newly connected to the implant (110), the identification process for all connected electrode leads (100) is carried out for every electrode lead one after the other, and **in that** the respective response signal is compared with the information on the electrode leads (100) that is already stored in a storage device of the implant and, based on the result of the comparison, the information of the newly connected electrode lead (100) is associated with the at least one socket (301) of the implant (110), this socket being connected with the new electrode lead (100).

4. An implant (110) with an electrode lead (100),
The electrode lead (100) comprising a plug (102), at least one electrical conductor (101), and an insulating tube (130) that insulates the at least one electrical conductor (101), a hermetically sealed passive RFID label (104) being embedded in the insulating tube (130) and/or in the plug (102) or in an insulating body of a separate accessory that is connectable with the insulating tube (130) or the plug (102), the RFID label (104) having an RFID chip (200) and an inlay antenna (210) that has an electrically conductive connection with the RFID chip (200), the inlay antenna (210) being electromagnetically coupled with the at least one electrical conductor (101) and the communication antenna (111),
the implant (110) further having a housing, the housing having a controller (120) arranged in it that is connected with a communication antenna (111),
the implant (110) having a socket (301), the plug (102) of the electrode lead (100) being inserted into the socket (301) of the implant (110), so that the electrical conductor (101) is electrically connected with the controller (120),
**characterized in that**
the controller (120) is set up to process an electromagnetic response signal of a bipolar antenna consisting of the communication antenna (111) and the electrical conductor (101) of the electrode lead (100).

5. An implant according to claim 4, **characterized in that** the controller (120) is set up to produce an electromagnetic interrogation signal, which is transmitted by a bipolar antenna consisting of the communication antenna (111) and the electrical conductor (101) of the electrode lead (100), the plug (102) of this electrode lead (100) being inserted into the socket (301), so that the electrical conductor (101) is electrically connected with the controller (120).

6. An implant according to one of the claims 4 through 5, **characterized in that** the socket (301) has multiple connections for connection with the multiple electrical conductors (101) of an electrode lead (100) or multiple electrode leads (100) and **in that** a transmission and/or receiving unit of the controller (120) is provided, this transmission and/or receiving unit being connected with the multiple connections through a multiplexer.

7. An implant according to any one of the claims 4 through 6, **characterized in that** the controller (120) is set up so that it produces the interrogation signal at regular and/or specifiable time intervals and/or after the occurrence of a predetermined event.

8. An implant according to claim 7, **characterized in that** the implant (110) has a detection unit (119) that detects the connection of an electrode lead (100) to the implant (110).

9. An implant according to claim 7 or 8, **characterized in that** the implant (110) has a detection unit (119) that detects the connection of an electrode lead (100) to the implant (110) by measuring the impedance of the respective connections for the electrode leads on the implant.

## Revendications

1. Procédé d'identification d'une ligne d'électrode (100) au moyen d'un implant (110), dans lequel la ligne d'électrode (100) présente une prise (102), au moins un conducteur électrique (101) et un tuyau d'isolation (130) isolant l'au moins un conducteur électrique (101), où une étiquette RFID (104) passive, fermée hermétiquement est incorporée dans le tuyau d'isolation (130) et/ou dans la prise (102) ou dans un corps isolant d'une partie complémentaire séparée pouvant être reliée avec le tuyau d'isolation (130) ou la prise (102), où l'étiquette RFID (104) présente une puce RFID (200) et une antenne d'incrustation (210) reliée de manière électriquement conductrice avec la puce RFID (200), où l'antenne d'incrustation (210) est couplée de manière électromagnétique avec l'au moins un conducteur électrique (101) et l'antenne de communication (111) ;
dans lequel l'implant (110) présente un dispositif de commande (120) et une antenne de communication (111) reliée avec le dispositif de commande ;
où l'implant (110) est relié avec la ligne d'électrode (100),
comprenant les étapes suivantes :
- l'envoi d'un signal d'interrogation électromagnétique par le conducteur électrique (101) devant être sélectionné et l'antenne de communication (111), où l'antenne de communication (111) et le conducteur électrique (101) sont en fonctionnement conjoint en tant qu'antenne bipolaire, où le signal d'interrogation est généré par le conducteur électrique (101) devant être sélectionné et le dispositif de commande (120) relié à l'antenne de communication (111),
- l'activation de l'étiquette RFID (104) par la réception du signal d'interrogation au moyen de l'antenne d'incrustation (210) de l'étiquette RFID (104) et la transmission du signal d'interrogation reçu à la puce RFID (200),
- le traitement du signal d'interrogation reçu par la puce RFID (200), la création d'un signal de réponse électromagnétique correspondant par la puce RFID (200) et l'envoi du signal de réponse au moyen de l'antenne d'incrustation (210) de l'étiquette RFID (104),
- la réception du signal de réponse par l'antenne bipolaire constituée de l'antenne de communication (111) et du conducteur électrique (101) devant être sélectionné de la ligne d'électrode (100), et
- le traitement du signal de réponse transmis reçu dans le dispositif de commande (120) vers le dispositif de commande (120).

2. Procédé selon la revendication 1, **caractérisé en ce que** la création du signal d'interrogation a lieu par le dispositif de commande (120) à des intervalles de temps réguliers et/ou pouvant être définis, et/ou après la survenue d'un évènement prédéfini, de préférence après le raccordement d'une ligne d'électrode (100) sur l'implant (110).

3. Procédé selon l'une des revendications 1 ou 2, **caractérisé en ce que**, lorsque deux ou plus de deux lignes d'électrode (100) sont raccordées à l'implant (110), où parmi ces lignes d'électrodes (100) une ligne d'électrode est nouvellement raccordée à l'implant (110), le procédé est effectué pour l'identification de toutes les lignes d'électrodes (100) raccordées les unes après les autres pour chaque ligne d'électrode et que le signal de réponse respectif est comparé avec des informations concernant les lignes d'électrodes (100) déjà stockées dans un dispositif de stockage de l'implant et en se basant sur le résultat de la comparaison, une association des informations de la ligne d'électrode (100) nouvellement raccordée a lieu avec l'au moins une douille (301) de l'implant (110) reliée avec la nouvelle ligne d'électrode (100).

4. Implant (110) doté d'une ligne d'électrode (100),
dans lequel la ligne d'électrode (100) présente une prise (330), au moins un conducteur électrique (101) et une tuyau d'isolation (130) isolant l'au moins un conducteur électrique (101), où une étiquette RFID (104) passive, fermée hermétiquement est incorporée dans le tuyau d'isolation (130) et/ou dans la prise (102), ou dans un corps isolant d'une partie complémentaire séparée pouvant être reliée avec le tuyau d'isolation (130) ou la prise (102), où l'étiquette RFID (104) présente une puce RFID (200) et une antenne d'incrustation (210) reliée de manière électriquement conductrice avec la puce RFID (200), où l'antenne d'incrustation (210) est couplée de manière électromagnétique avec l'au moins un conducteur électrique (101) et l'antenne de communication (111) ;
où l'implant (110) présente en outre un boîtier, où un dispositif de commande (120), lequel est relié avec l'antenne de communication (111), est disposé dans le boîtier, où l'implant (110) présente une douille (301), où la ligne d'électrode (100) est branchée avec sa prise (102) dans la douille (301) de l'implant (110) de sorte que le conducteur électrique (101) est relié électriquement avec le dispositif de commande (120),
**caractérisé en ce que**
le dispositif de commande (120) est prévu pour le traitement d'un signal de réponse électromagnétique d'une antenne bipolaire constituée d'une antenne de communication (111) et du conducteur électrique (101) de la ligne d'électrode (100).

5. Implant selon la revendication 4, **caractérisé en ce que** le dispositif de commande (120) est conçu pour la génération d'un signal d'interrogation électromagnétique, lequel est envoyé par une antenne bipolaire constituée d'une antenne de communication (111) et du conducteur électrique (101) de la ligne d'électrode (100), où cette ligne d'électrode (100) est branchée avec sa prise (102) dans la douille (301) de sorte que le conducteur électrique (101) est relié électriquement avec le dispositif de commande (120).

6. Implant selon l'une des revendications 4 à 5, **caractérisé en ce que** la douille (301) présente une multiplicité de connecteurs pour la liaison avec une multiplicité de conducteurs électriques (101) d'une ligne d'électrode (100) ou de plusieurs lignes d'électrodes (100) et qu'une unité de transmission et/ou de réception du dispositif de commande (120) est prévue, laquelle est reliée avec la multiplicité de connecteurs par le biais d'un multiplexeur.

7. Implant selon l'une des revendications 4 à 6, **caractérisé en ce que** le dispositif de commande (120) est conçu de telle manière qu'il génère le signal d'interrogation à des intervalles de temps réguliers et/ou pouvant être prédéfinis, et/ou après la survenue d'un évènement prédéfini.

8. Implant selon la revendication 7, **caractérisé en ce que** l'implant (110) présente une unité de détection (119), laquelle détecte le raccordement d'une ligne d'électrode (100) sur l'implant (110).

9. Implant selon la revendication 7 ou la revendication 8, **caractérisé en ce que** l'implant (110) présente une unité de détection (119), laquelle détecte le raccordement d'une ligne d'électrode (100) sur l'implant (110) par la mesure de l'impédance des connexions respectives pour les lignes d'électrodes sur l'implant.
